Europäisches Patentamt

European Patent Office

Office européen des brevets

(11) Publication number: **0 349 173**
**A1**

## (12) EUROPEAN PATENT APPLICATION

(21) Application number: **89306170.5**

(22) Date of filing: **19.06.89**

(51) Int. Cl.4: **A61F 2/38**

(30) Priority: **22.06.88 GB 8814863**

(43) Date of publication of application:
**03.01.90 Bulletin 90/01**

(84) Designated Contracting States:
**AT BE CH DE ES FR GR IT LI LU NL SE**

(71) Applicant: **Polyzoides, John**
**108 School Road Hockley Heath**
**Solihull West Midlands(GB)**

Applicant: **Tsakonas, Athanassis**
**5 Agiou Serafim Analypsy**
**Thessaloniki(GR)**

(72) Inventor: **Polyzoides, John**
**108 School Road Hockley Heath**
**Solihull West Midlands(GB)**
Inventor: **Tsakonas, Athanassis**
**5 Agiou Serafim Analypsy**
**Thessaloniki(GR)**

(74) Representative: **Spruce, George Philip et al**
**Shaw, Bowker & Folkes Whitehall Chambers**
**23 Colmore Row**
**Birmingham B3 2BL(GB)**

(54) **Knee prosthesis.**

(57) Knee joint prosthesis comprising a metal or other femoral component (10) providing substitute condylar bearing surfaces (20); a metal or other tibial component (12) whose major portion (24) provides a platform; and a single plastics or other meniscal element (14) seating on said platform and engaged by the said bearing surfaces, the tibial component and the meniscal element being provided with inter-engaging locating means, typically a pivot (32) which locates in a shaped central cut-away (44) of the element and a locating flange (36) co-acting with a recess (46) in the front edge of the element, to prevent excessive displacement while allowing limited rotary movement about the tibia axis together with limited forward and rearward gliding movement on articulation of the assembled joint.

Fig.1

## KNEE PROSTHESIS

This invention relates to knee joint prostheses.

The invention is particularly applicable to the treatment of patients having one or more damaged or worn bearing surfaces of their knee joints i.e. wear or damage of the bearing surfaces of the femur, tibia and/or associated meniscii, but the cruciate ligaments of the knee are healthy and their retention is desirable. Said wear or damage may be to due to primary or secondary osteoarthritis, rheumatioid arthritis, inflammatory connective tissue disease and/or due to or aggravated by obesity, over-working or stress on the joint, and/or previous trauma of the joint, where the torsional stresses are transmitted to the femur and tibia eroding the articular cartilage and exposing the bone.

Various forms of knee prosthesis are known. Many of them operate by replacing not only the bearing surfaces of the bone and cartilage but also the associated ligaments thus necessitating more extensive surgery than is strictly necessary, or precluding operative treatment until it is judged that the condition justifies the more drastic substitution.

In a normal healthy knee joint, in movement from a sitting to a standing position, the femur rotates medially relative to the tibia to lock the knee in extension. In flexion, for example when climbing stairs the meniscii glide backwards as well as moving to accommodate said medial rotation. Many of the known prostheses, while replacing the bearing surfaces of the knee joint, do not provide for or allow medial rotation and gliding movement with the result that excessive loading, particularly torsional forces are transmitted to the interfaces between the implant components and the bones with consequent risk of breakage or loosening.

It is also known to provide a knee prosthesis which replaces the bone and cartilage bearing surfaces and retains the cruciate ligaments while attempting to provide the range of articulation of the natural knee, including some degree of rotation about the medial axis. One example of this latter type of prothesis is that designed by us, the present applicants, and manufactured and sold by Zimmer Limited known as the "Gliding Meniscal Knee".

The object of the present invention is to provide an improved knee joint prosthesis which replaces the bearing surfaces of the knee, incorporates a minimum number of components, is economical to manufacture and implant, which is readily adaptable to the various needs of different patients both young and old, and which it is believed will be particularly reliable, effective, wear-resistant, and long-lasting.

According to the invention there is provided a knee joint prosthesis comprising a femoral component to be operatively anchored to the prepared end of a subject's femur and shaped to provide a pair of bearing surfaces acting in substitution for the respective femoral condyles; a tibial component to be operatively anchored to the prepared end of the subjects's tibia and shaped to provide a thrust plateau facing the femoral component in use; and a single meniscal element seated on said plateau in use for coacting engagement by both said bearing surfaces of the femoral component, the tibial component and said element being provided with interengaging locating means resisting excessive displacement of one relative to the other but permitting both limited rotary movement of said element about the anatomical medial longitudinal axis of the tibia and limited forward and rearward gliding movement thereof during and in response to relative articulation of the said components with the associated bones in use.

Preferably said components are formed from metal, e.g. a cobalt chrome alloy, and the meniscal element is formed from a high duty low friction plastics material, e.g. ultra high molecular weight polyethylene ( U H M W P).

Either or both said components may be secured to the respective prepared end or ends of the bone or bones by use of a cement in known manner with or without the use of mating complementary locating formations such as pegs or spigots located in sockets formed in the bone or bones. The interface between the component and the bone may be provided with a coating or surface finish of known kind which is believed to encourage bonding by ingrowth of the bone tissue.

However, in the present state of the art, indications are that cement and/or bone ingrowth surface finishes are not entirely successful or reliable, particularly in the long term e.g. where prostheses are implanted in young and active subjects with substantial life expectancy.

For the latter reason it is preferred that both (or at least one) of said components are anchored to the end or ends of the associated bones by non-cemented means, particularly as defined in the following paragraph.

According to another aspect of the invention means for anchoring a prothesis or a component of a prosthesis to a prepared end or other part of a subject's bone comprises at least one spigot or peg of the prosthesis or component which is operatively press fitted into a mating socket cut in the bone, said spigot or peg having one or more longitudinal slots open to its distal end so that it is divided into two or more spaced parallel limbs and also having a series of peripheral grooves in at least a substantial portion of its length to define a plurality of laterally directed ribs for gripping engagement with the bone socket, said limbs being capable of resilient deformation towards each other by said press fitting to increase the grip in the socket, said ribs and grooves possibly encouraging additional anchorage by subsequent growth of the bone into said grooves and/or slots.

Said spigots or pegs may be formed of metal, for example a titanium alloy. While they may be formed integrally with remainder of the associated prosthesis or component if the latter is formed from the same material; conveniently they are mounted thereon e.g. by having a stem which is received in an aperture in said remainder secured, for example, by welding if both the spigot and the rest of the prosthesis or component are of a metal alloy.

If said anchoring means is applied to the preferred form of knee joint prosthesis according to the invention conveniently two spaced parallel anchoring pegs will be provided on each of the tibial and femoral components. If the components are formed of a cobalt chrome alloy the associated anchoring pegs will preferably be of titanium alloy and be welded to the main bodies of said components as referred to above.

It is also preferred that the knee joint prosthesis of the invention is shaped to accommodate the natural cruciate ligaments of the subject and thus does not require any constraining or stabilising mechanism to retain the components in correct relationship through the range of required articulation.

An embodiment of the invention is now more particularly described with reference to the accompanying drawings wherein

Figure 1 is a side view of a femoral component,

Figure 2 is a front view thereof,

Figure 3 is a section on line B-B of Figure 2,

Figure 4 is a section on line A-A of Figure 1

Figure 5 is a plan view of an associated tibial component,

Figure 6 is a part sectioned rear view of the latter component,

Figure 7 is a side view of the latter component,

Figure 8 is a plan view of an associated meniscal element,

Figure 9 is a rear elevation of the latter component,

Figure 10 is a side view of the latter component,

Figure 11 is a part sectional side elevation of an anchoring peg of said components, and

Figure 12 is an end view of said peg.

The knee prosthesis embodying the invention shown in the drawings consists of three parts only, a femoral component 10, a tibial component 12, and a meniscal element 14 which is operatively interposed between said components to form the assembled joint, the subject's cruciate ligaments being retained and accommodated by the shaping of the joint.

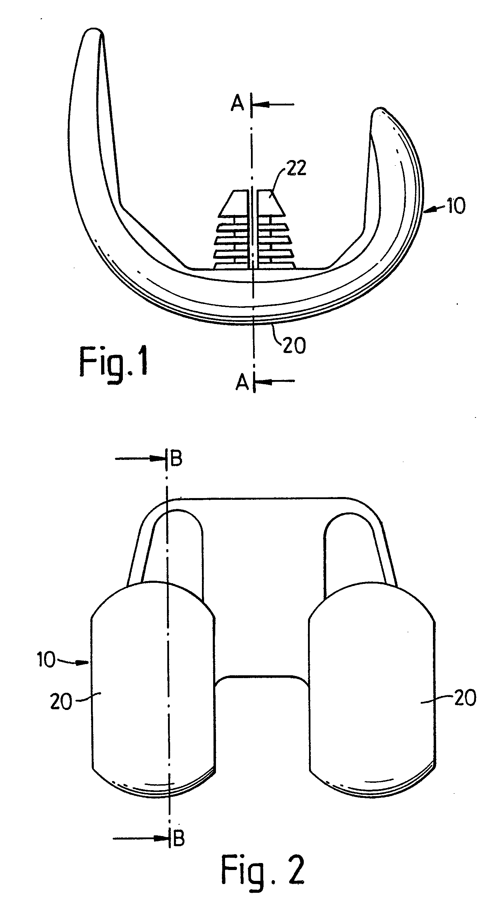

Referring firstly to Figures 1-4 the femoral component 10 is formed from metal, typically cobalt chrome alloy and is of generally conventional shape for operative fitting to the prepared end of the subject's femur for total replacement of the condylar surfaces of the bone. The component provides two highly polished substitute condylar bearing surfaces 20 having compound curvature to provide a full range of natural knee movement.

In the preferred form shown component 10 is secured to the end of the femur by anchoring means in the form of a pair of anchor pegs 22 which will be described in greater detail below.

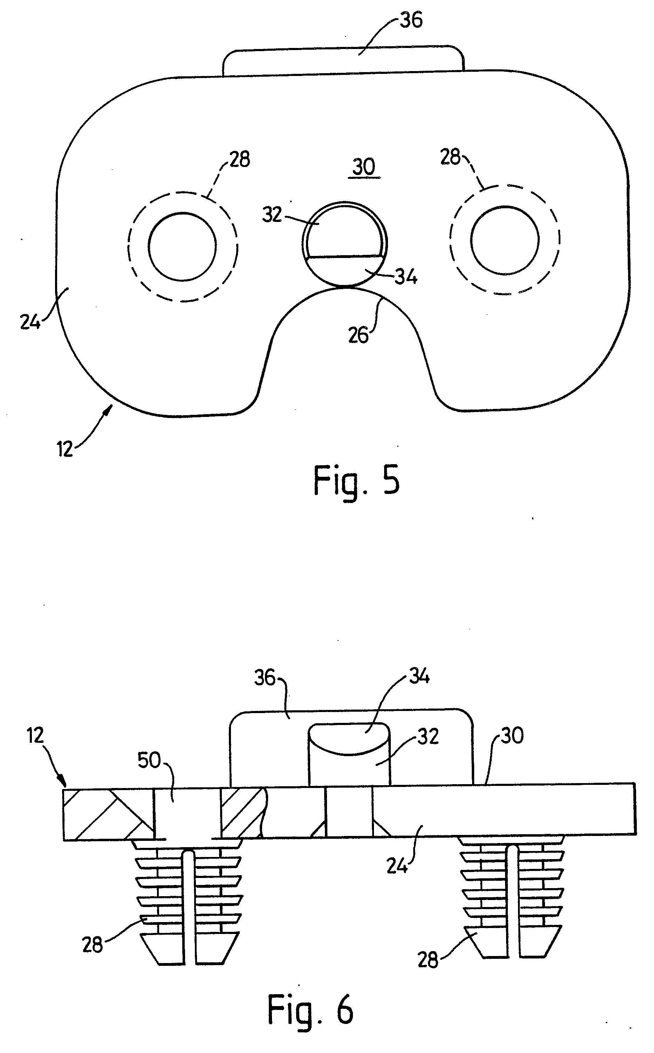

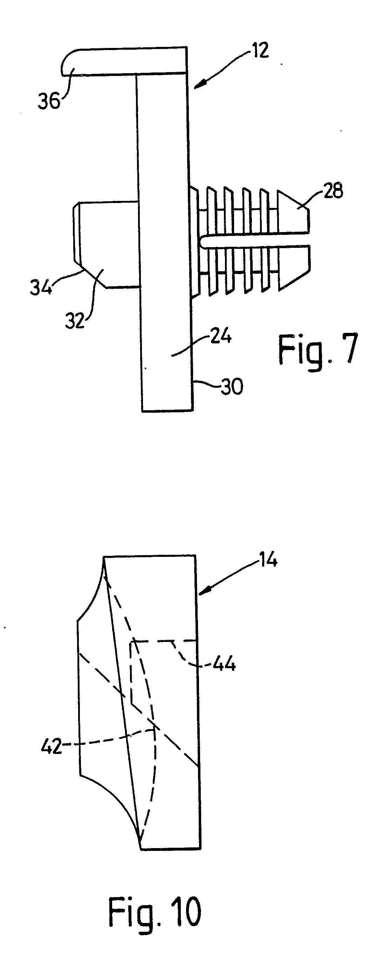

The tibial component 12 shown in Figures 5-7 is also formed from metal, typically cobalt chrome alloy, its major portion 24 being a planar platform which is operatively secured to a prepared flat end face of the upper end of the subject's tibia so that it extends generally horizontally when the tibia is upright. The corners of portion 24 are radiused and a gap 26 is defined in the rear edge in corresponding relationship to the gap between the condylar bearing portions of the femoral component.

Component 12 is again operatively secured to the respective bone end by a pair of anchor pegs 28.

The flat upper face 30 of portion 24 is given a smooth finish, this face serving as a thrust supporting plateau facing the femoral component 10 in use. At the centre of said face midway between the axes of the pegs 28 is an upwardly projecting cylindrical pivot 32, the innermost part of the radiused edge defining gap 26 is in immediate proximity to the periphery of pivot 32 and its upper rear edge is chamfered at 34 to provide clearance in this region.

The forward edge of portion 24 is provided with an upwardly extending transverse rectilinear locating flange 36 centred in line with pivot 32 and having a width approximating to the spacing between the anchor pegs 28.

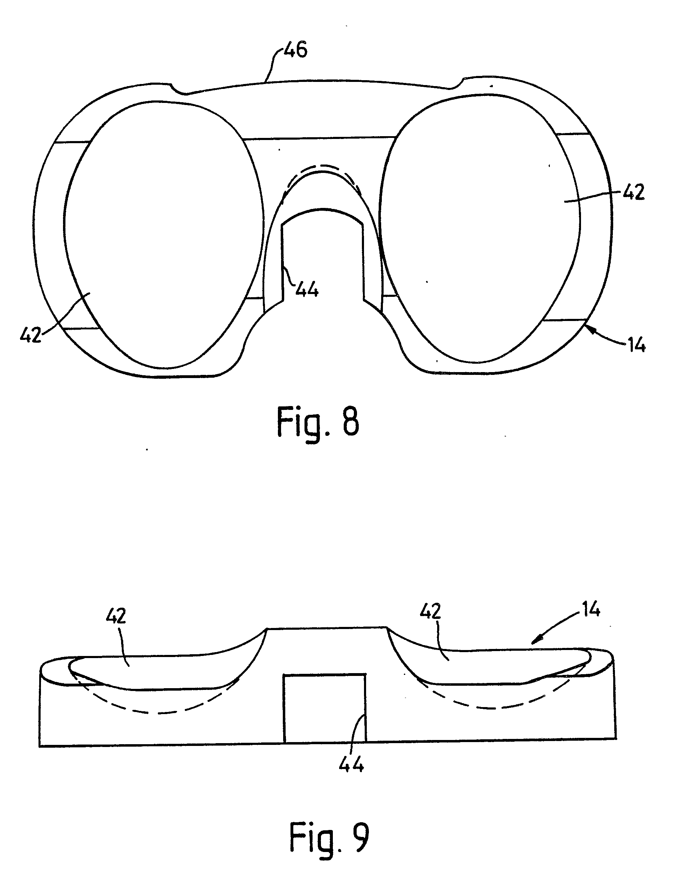

The meniscal element 14 shown in Figures 8-10 is formed from an inert low friction heavy duty plastics material, for example U H M W P and is in the form of a pad which is operatively interposed between components 10 and 12. The underface of element 14 is flat for sliding engagement with the upper face 30 of component 12 and the shape of element 14 in plan view corresponds generally to that of major portion 24 of that component.

The upper face of element 14 defines a pair of spaced concave hollows 42 in which the condylar bearing surfaces 20 of component 10 seat for low friction angular movement about a lateral axis of the assembled joint during flexion and extension.

The mating engagement between said hollows and said bearing surfaces will also shift element 14 about a medial axis longitudinally of the associated tibia by sliding movement of element 14 on the upper face 30 of component 12. Displacement of element 14 relative to component 12 is limited in the assembled joint by interengagement of their locating means i.e. pivot 32 locates in a shaped cutaway 44 centrally of element 14 midway between the hollows 42, and the locating flange 36 of component 12 coacts with a radiused shallow recess 46 (Figure 8) in the front edge of element 14, the effective length of this recess limiting the rotary movement of element 14 on face 30. The latter movement is generally centred on the pivot 32 though possibly with some latitude depending upon the clearance allowed between said pivot and the coacting cutaway 44. Depending on the needs of the subject the degree of rotary movement will be selected by the use of appropriate sets of components; typically a range of rotary movement of 10° to 15° will be provided.

This facility for rotary movement accommodates the natural articulation of the knee joint including its degree of rotation about the anatomical medial axis of the tibia during knee flexion.

The effective minimum depth of element 14 between the innermost end of cutaway 44 which coacts with pivot 32 and the inner radiused face of recess 46 is substantially less than the space between pivot 32 and the flange 36 of component 12. Thus, as well as the limited rotational freedom of element 14 relative to component 12 there is a predetermined limited freedom for forward and rearward gliding movement of element 14 during flexion and extension of the joint, so providing to such preselected extent as may be desirable, the full range of articulation of the natural knee.

This freedom of movement, particularly said rotational freedom, also gives a self-aligning effect in that it will compensate for any lack of exact positioning of the prosthesis on implantation particularly as the operation has to be carried out with the knee in a bent position so that the desired alignment cannot be effectively determined with precision.

The articulation of the prosthesis is such that excessive stressing of the anchorage of components 10 and 12 to the bone is avoided and the full range of movement is provided controlled by the natural ligaments and other soft tissue of the total joint following implantation of the prosthesis.

It will be appreciated that a range of sizes of the components 10 and 12 will be made available so that the dimensions most appropriate to the subject can be selected, for example four sizes; small, standard, larger standard, and large may be provided. A range of elements 14 of varying geometry and dimensions, e.g. effective thickness, may also be provided, again for selection according to the most desirable geometry and range of articulation of the implanted joint for a particular subject e.g. a range of heights from 4mm to 12mm enabling the collateral ligaments to be tensed to any required degree as well as correction of the joint in other ways.

Thus the facility for ready selection and substitution of meniscal elements 14 from a range of sizes and characteristics provides a very simple facility for correcting deformities of the knee joint in the course of the operative treatment, for example correction of varus or valgus by selecting an element 14 which is thicker on one side than the other. Rotary malalignment which is a common feature of the severely deformed knee can also be corrected by use of the prosthesis.

The simple three-part assembly and the absence of any constraining or stabilising mechanism in the prosthesis beyond the basic inter-engagement between the said three parts makes the joint easy to implant with the aid of appropriate instruments and jigs as known in the art for preparing the bone ends etc., and, it is believed, will provide comfortable and trouble free long term service. If any revision should be needed the simple construction also facilitates further operative treatment or correction, for example the meniscal element 14 is readily replaceable. A patella implant, typically also formed from a plastics material, may be used in conjunction with the prosthesis if required.

As previously referred to, the components 10 and 12 may be anchored to the associated bones by various means or combinations of means including those already known in the art, for example by cementing whether or not in conjunction with locating pegs or spigots, and/or surface finishes etc aimed at encouraging bone ingrowth.

However, the preferred means of securing said components does not place reliance on cementing and/or bone ingrowth as the results of these methods, particularly in the long term, are not regarded as fully proved and/or reliable.

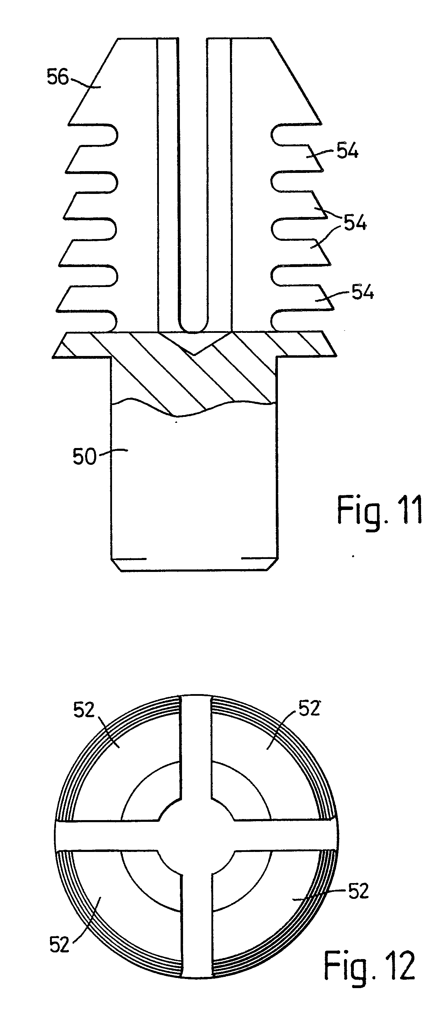

Figures 11 and 12 show details of the preferred form of anchor pegs 22,28 of the components 10,12.

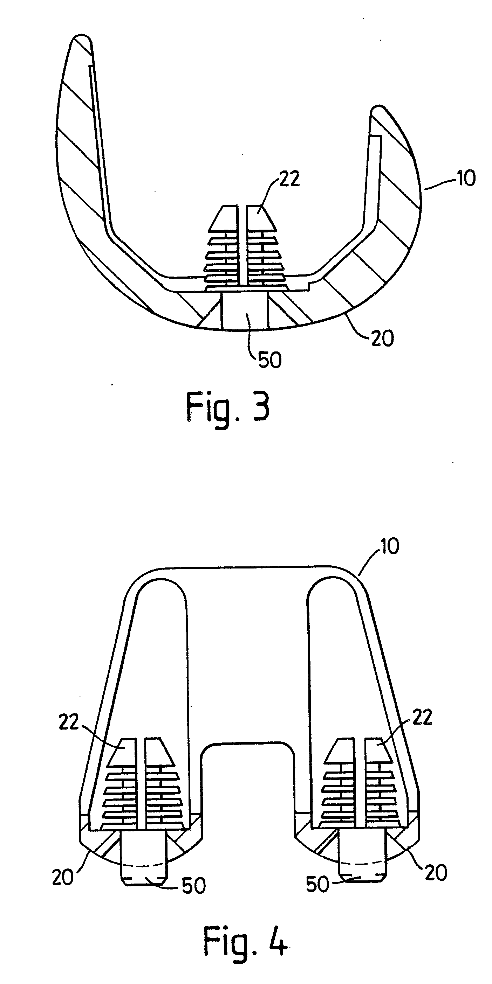

Each peg is formed from metal. A titanium alloy is preferred as being one of the most bio-inert materials for chrome cobalt alloys have been known to produce a reaction with bone, and bone ingrowth has proved to be better with titanium alloy than chrome cobalt alloy. In this example the peg has a cylindrical stem 50 which is fitted into a corresponding through bore of the major part of the respective component as seen in Figures 3,4 and 6, the mouth of said bore on the side opposite to the projection of the peg being countersunk for a securing weld joint to be formed which is subsequently smoothed and flattened. Figure 4 shows pegs 22 in position with their stems 50 projecting from the component 10 prior to welding, after which excess length of the stem will be removed and the component will be finished to the condition shown in Figure 3.

The remaining portion of the peg projecting beyond stem 50 is separated into four parallel spaced equiangular limbs 52 as best seen in Figure 12 by longitudinal slots extending from the distal end of the peg almost to stem 50.

The periphery of the acting part of the peg is also provided with a series of radial ribs 54 defined by peripheral grooves, in this example five in number.

The remaining distal end portion 56 is part-conical tapering to the distal end face at which its minimum diameter is substantially equal to the diameter at the full depth of said grooves.

This end portion provides a lead-in when the peg is pressed home into a socket of predetermined diameter cut in the prepared bone end. Limbs 52 can flex inwardly toward each other during said insertion so that they are in gripping engagement with the bone socket and further security is against forces tending to withdraw the peg provided by ribs 54 each tapering in diameter toward said distal end to form a saw-tooth profile as viewed in axial section (see Figure 11).

This form of anchoring peg provides immediate effective security which will withstand substantial stresses without reliance on cement and without awaiting bone growth. It is possible that some bone growth may take place with the passage of time into said grooves and/or longitudinal slots of the pegs, if so this will provided added security.

It is anticipated that the above described form of anchoring peg may have applications for securing prostheses other than the knee joint of the invention, for example other forms of knee joint prosthesis and/or prostheses for joints other than the knee or any other application where a component has to be secured to a bone.

## Claims

1. A knee joint prosthesis comprising a femoral component (10) to be operatively anchored to the prepared end of a subject's femur and shaped to provide a pair of bearing surfaces (20) acting in substitution for the respective femoral condyles; a tibial component (12) to be operatively anchored to the prepared end of the subjects's tibia and shaped to provide a thrust plateau (30) facing the femoral component in use; and meniscal element means seated on said plateau in use for coacting engagement by said bearing surfaces of the femoral component; characterised in that said meniscal element means is a single meniscal element (14) for engagement by both said bearing surfaces, and in that the tibial component and said element are provided with interengaging locating means (32, 44; 36) resisting excessive displacement of one relative to the other but permitting both limited rotary movement of said element about the anatomical medial longitudinal axis of the tibia and limited forward and rearward gliding movement thereof during and in response to relative articulation of the said components with the associated bones in use.

2. A prosthesis as in Claim 1 characterised in that said components (10, 12) are formed from metal.

3. A prosthesis as in Claim 2 characterised in that the metal is a cobalt chrome alloy or a titanium alloy.

4. A prosthesis as in Claim 1, 2 or 3 characterised in that the meniscal element (14) is formed from a high duty low friction plastics material.

5. A prosthesis as in Claim 4 characterised in that said material is ultra high molecular weight polyethylene.

6. A prosthesis as in any preceding claim characterised by being shaped to accommodate the natural cruciate ligaments of the subject.

7. A prosthesis as in any preceding claim characterised in that at least one of said components (10, 12) includes a locating formation (22, 28) for location in a complementary socket formed in the prepared end of the respective associated bone in use.

8. A prosthesis as in Claim 7 characterised in that said locating formation or formations (22, 28) are each adapted to anchor the respective component to the associated bone in use without the use of a cement.

9. A prosthesis as in Claim 7 or Claim 8 characterised in that the or each locating formation (22, 28) is a spigot or peg of the respective component which is operatively press-fitted into the mating socket of the associated bone, said spigot or peg having one or more longitudinal slots open to its distal end so that it is divided into two or more spaced parallel limbs (52) and also having a series of peripheral grooves in at least a substantial portion of its length to define a plurality of laterally directed ribs (54) for gripping engagement with said socket, the limbs being capable of resilient deformation towards each other by said press-fitting to increase the grip in the socket.

10. A prosthesis as in Claim 7, 8 or 9 characterised in that the or each locating formation (22, 28) is formed from titanium alloy.

11. A prosthesis as in Claims 9 or 10 characterised in that the or each locating formation (22, 28) has a part-conical distal end portion.

12. A prosthesis as in Claim 9, 10 or 11 characterised in that the ribs (54) of the or each spigot or peg each taper in diameter towards the distal end of the spigot or peg to form a saw-tooth profile as viewed in axial section.

13. A prosthesis as in any preceding claim characterised in that surfaces of the components (10, 12) which will form interfaces with the associated bone in use are pro vided with a coating or surface finish intended to encourage bonding by ingrowth of bone tissue therewith.

14. A prosthesis as in any preceding claim characterised in that the meniscal element (14) is shaped to correct previously existing deformities of the subjects's knee joint.

15. A prosthesis as in Claim 14 characterised in that the meniscal element (14) is thicker on one side than the other for correction of varus or valgus.

Fig.1

Fig. 2

Fig. 3

Fig. 4

Fig. 5

Fig. 6

Fig. 7

Fig. 10

Fig. 8

Fig. 9

Fig. 11

Fig. 12

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 301 553 (NOILES)<br>* Column 9, lines 10-34; column 8, lines 44-56; figures 9,10; column 3, lines 46-49; column 6, lines 38-42 * | 1-7 | A 61 F    2/38 |
| Y | | 8-15 | |
| Y | FR-A-2 615 726 (LANDOS)<br>* Abstract; figures; page 2, lines 19-29; claims 13-16 * | 8-12 | |
| Y | US-A-4 714 473 (BLOEBAUM)<br>* Abstract; figures * | 13 | |
| Y | JOURNAL OF BONE AND JOINT SURGERY, vol. 5A, no. 7, September 1983, pages 906-919, Boston, Massachusetts, US; J.-M. CLOUTIER: "Results of total knee arthroplasty with a non-constrained prosthesis"<br>* Pages 906-907; figure 1-B * | 14-15 | |

**TECHNICAL FIELDS SEARCHED (Int. Cl.4)**

A 61 F

The present search report has been drawn up for all claims

| Place of search | Date of completion of the search | Examiner |
|---|---|---|
| THE HAGUE | 25-09-1989 | SANCHEZ Y SANCHEZ J. |